# EUROPÄISCHE PATENTSCHRIFT

(11) **EP 0 507 968 B1**
(45) Veröffentlichungstag und Bekanntmachung des Hinweises auf die Patenterteilung: **06.09.1995**
(21) Anmeldenummer: 91105463.3
(22) Anmeldetag: 06.04.1991
(51) Int. Cl.: C12P 21/04, C12N 1/14

(54) **Verfahren zur fermentativen Herstellung und Isolierung von Cyclosporin A sowie neue Cyclosporin- bildende Stämme**
Method for the fermentative production and isolation of cyclosporin A and new cyclosporin forming strains
Procédé de production par fermentation et isolement de cyclosporine A ainsi que de nouvelles souches productrices de cyclosporine

(43) Veröffentlichungstag der Anmeldung: 14.10.1992
(73) Patentinhaber: ARZNEIMITTELWERK DRESDEN GmbH, 01445 Radebeul (DE)
(72) Erfinder: Rudat, Wolf-Rüdiger Dr., O-8054 Dresden (DE); Bormann, Ernst-Joachim Dr., O-6902 Jena (DE); Arnold, Günter Dr., O-5300 Weimar (DE)

(56) Entgegenhaltungen:
- EP-A- 379 708

## Beschreibung

Die vorliegende Erfindung betrifft ein Verfahren zur Herstellung des zyklischen Oligopeptides Cyclosporin A.
Insbesondere bezieht sich die Erfindung auf ein mikrobiologisches Verfahren unter Verwendung neuer Produzentenstämme, sowie die Isolierung und Reingewinnung des Wirkstoffes.

Cyclosporin A gehört zu einer Gruppe von zyklischen Undekapeptiden mit entzündungshemmenden, immunsupressiven, antifungalen und antiparasitischen Eigenschaften. Von besonderem Interesse für die Humanmedizin haben sich die immunmodulatorischen Eigenschaften erwiesen, die in der Transplantationschirurgie und der Behandlung von Autimmunerkrankungen genutzt werden.

In den Patentschriften CH-PS 589716 und CH-PS 603790 wird diese neuartige Substanzklasse, bezeichnet als Cyclosporine, erstmals erwähnt und in der Monographie von BOREL (Ciclosporin, Progress in Allergy 38, Karger Verlag, 1986) hinsichtlich ihrer chemischen Struktur und biologischen-pharmakologischen Wirkung umfassend beschrieben.

Es ist bekannt,daß in Herstellungsverfahren für Cyclosporin A die Fähigkeit von Mikroorganismen zur Synthese der natürlicherweise vorkommenden Cyclosporine genutzt wird, indem man bestimmte Spezies von Mikroorganismen als Produktbildner in einem Fermentationsverfahren bei Einhaltung allgemein üblicher Kulturbedingungen in Nährlösungen, die komplexe Substrate enthalten, kultiviert und in ebenso bekannter Weise den Wirkstoff isoliert und reindarstellt.

In ausgedehnten Screeninguntersuchungen wurden zahlreiche Pilzspezies mit Cyclosporin-Bildungsvermögen gefunden. DREYFUSS (Sydowia, Bd.39, 1986,22-36) beschreibt für die Pilzgattungen Cylindrocarpon und Fusarium eine ausschließlich emerse Cyclosporin-Bildungsfähigkeit, während für Stämme der Hyphenpilze Tolypocladium geodes, Trichoderma viride, Neocosmospora vasinfecta, Isaria felina, Verticillium spec., Acremonium spec. und Beauveria nivea (ursprünglich als Tolypocladium inflatum beschrieben) in Submerskulturen Cyclosporinbildung nachgewiesen wurde. Die höchste Produktivität des Wirkstoffes konnte mit Stämmen des Pilzes Beauveria nivea erreicht werden. In der großtechnischen Herstellung von Cyclosporin A hat daher dieser Stamm besondere Bedeutung erlangt. Nach Angaben von TRABER et al. (J. Antibiotics, Vol. 42, 1989, 591-597) produziert eine Hochleistungsmutante von Beauveria nivea (im Artikel als Mutante von Tolypocladium inflatum NRRL 8044 beschrieben) etwa 500 mg Cyclosporin A pro Liter. In einem chemisch definierten synthetischen Medium mit den Hauptkomponenten Saccharose und Apfelsäure als Kohlenstoffquelle und Ammoniumionen als Stickstoffquelle erreichten KOBEL und TRABER (Europ. J. Appl. Microbiol. Biotechnol. Vol. 14, 1982 237-240,) eine Ausbeute von 101 mg Cyclosporin A pro Liter in 14 Kulturtagen. Durch zusätzliche Fütterung des Produktbildners mit ausgewählten Aminosäuren in einer Konzentration von 8 g/l konnte das Ausbeuteniveau zwar verbessert werden, was jedoch für ein technisch relevantes Verfahren keinesfalls eine ökonomisch tragfähige Lösung darstellt.
Ähnliche Nachteile weist ein Fermentationsregime nach AGATHOS et al. (Ann. N. Y. Acad. Sci. 506, 1987, 657-662,) auf, welches eine Fermentation in einem halbsynthetischen Medium mit einer Ausbeute von 530 mg Cyclosporin A pro Liter nach 16 Kulturtagen beschreibt, wobei jedoch als Kohlenstoffquelle, die für ein technisches Verfahren als zu kostenintensiv eingeschätzte Maltose eingesetzt wird.

Im Sinne einer ökonomischen Nutzung der Produzentenstämme sind die beschriebenen Raum-Zeit-Ausbeuten für ein technisches Herstellungsverfahren äußerst unbefriedigend, welche sich zudem auf die zwangsläufig mit Wirkstoffverlusten verbundene Isolierung und Reinigung der verschiedenen Cyclosporine, insbesondere des Cyclosporin A, nachteilig auswirken.
Die beschriebenen Methoden zur Isolierung von Cyclosporin A aus Kultursuspensionen verschiedener Stämme tragen in allen Fällen der zu erwartenden Empfindlichkeit der Peptidbindungen im Cyclosporin-A-Molekül Rechnung und beginnen unter Vermeidung erhöhter Temperaturen mit einer flüssig-flüssig Extraktion der Kultursuspension oder einer Extraktion des abgetrennten Naßmycels [US-PS 4 117 118; US-PS 4 215 199; DE-PS 2 455 859; BE-PS 866 810; v.Warburg et al. Helv.Chim.Acta 55 (1976) 1075]. Die Extraktionsrückstände werden in den meisten Fällen zwischen 30%igem Methanol und Petroläther verteilt und das so vorentfettete Material an Kieselgel, Aluminiumoxid und/oder Sephadex LH 20 mehrfach chromatographiert, wobei zum Teil erhebliche Adsorbensmengen zum Einsatz kommen, die bis zum 1000-fachen des Substrates gehen.(DE-PS 2 455 859). Dieser hohe Reinigungsaufwand sowie der Zwang zur sofortigen Aufarbeitung der geernteten Kulturbrühe bei Naßmycelaufarbeitung sind als wesentliche Mängel der bekannten Technologie anzusehen. Als Nachteil der gegenwärtig bekannten Verfahren ist ferner der hohe Aufwand an Lösungsmitteln insbesondere solcher mit geringer Umweltverträglichkeit festzustellen.

Der Erfindung liegt die Aufgabe zugrunde, die Zahl der verfügbaren hochproduktiven Cyclosporinbildner zu erweitern und ein ökonomisch effizientes mikrobiologisches Verfahren zu beschreiben, welches gestützt auf den Einsatz dieser neuen Mikroorganismen, die Herstellung von Cyclosporin A in synthetischen Medien ohne den Einsatz komplexer Stickstoffsubstrate ermöglicht. Ferner war es Aufgabe der Erfindung ein technologisch vorteilhaftes ,umweltverträgliches und effizientes Isolierungs- und Reinigungsverfahren für Cyclosporin A mit guten Ausbeuten zu erreichen.

Dabei sollte der Verbrauch an Hilfsstoffen möglichst klein gehalten und auf umweltverträgliche Stoffe begrenzt werden.

In Übereinstimmung mit der vorliegenden Erfindung wurde aus einem natürlichen Habitat der subtropischen Klimazone ein neuer, bisher unbekannter Mikroorganismus isoliert und rein kultiviert, der nicht nur die gesuchte Stoffgruppe als Metabolit akkumuliert, sondern diese und insbesondere das Cyclosporin A nach entsprechender Stammkonditionierung in Folge von Mutagenese- und Selektionsprozessen unter submersen Kulturbedingungen im Rahmen eines mehrstufigen technischen Fermentationsverfahrens in einer völlig unerwartet hohen Größenordnung bildet. Dieses Ergebnis ist insbesondere deshalb überraschend, da bisher chemisch definierte vollsynthetische Medien für die Wirkstoffsynthese einer Produktionskultur in diesen Größenordnungen als nicht geeignet erschienen.

Bei dem originellen Mikroorganismus handelt es sich um einen Vertreter der imperfekten Hyphenpilze, der aus einer Bodenprobe aus der Sierra del Rosario (Kuba) isoliert und taxonomisch einer neuen Gattung und Art mit der Bezeichnung Sesquicilliopsis rosariensis G.ARNOLD zugeordnet wurde.

Der Organismus wächst auf verschiedenartigen Nährböden, welche die üblichen Nährstoffe für Pilze enthalten. Bei Anzucht auf Malz-Agar im natürlichen Tag-Nacht-Rhythmus entstehen innerhalb von 10 Tagen ca. 8 cm große Kolonien mit schwach ausgebildetem, leicht flockigem, weißen Luftmyzel, welches sich bis zum Kolonierand erstreckt. Eine Ringbildung ist nur schwach in den Außenpartien der Kolonie zu beobachten. Ein schwacher Geruch nach Suppenwürze ist feststellbar. Das Luftmyzel besteht aus septierten, verzweigten Hyphen, welche in Masse weiß erscheinen. Die aufsteigenden bis aufrechten Konidienträger sind in der Regel stark und mehr oder weniger wirtelig oder unregelmäßig verzweigt, tragen an ihren Zweigenden mehrere, durch Übergipfelung entstehende Phialiden, die farblos, pfriemlich und schlank gestaltet sind. Die Konidien werden zu mehreren nacheinander an der Spitze der Phialiden gebildet; sie sind länglich, einzellig, hyalin, glatt- und dünnwandig, zu einem kleinen Köpfchen verklebt. Chlamydosporen wurden nicht beobachtet.

Von allen ähnlichen Gattungen mit phialidischen konidiogenen Zellen (z. B. mikrokonidienbildende Fusarien, Gliocladium, Verticillium, Sesquicillium, Sympodiophora) unterscheidet sich der Stamm Sesquicilliopsis rosariensis G. ARNOLD infolge genannter Besonderheiten, vor allem aber aufgrund der Art und Weise der Anordnung der konidiogenen Zellen.

Der neue Mikroorganismus, der im vorliegenden Verfahren zur Herstellung von Cyclosporinen verwendet wird, ist gemäß dem Budapester Vertrag bei der DSM -Deutsche Sammlung von Mikroorganismen und Zellkulturen GmbH D-38124 Braunschweig, Bundesrepublik Deutschland, unter der Nummer IMET 43888 hinterlegt; ebenso ist die hochproduktive Stammlinie F 605 hinterlegt, welche die Hinterlegungsnummer IMET 43887 erhalten hat. Dabei umfaßt die Erfindung natürlicher Weise auch alle anderen durch Mutation und/oder Selektion des Wildstammes oder der obengenannten Mutante gezüchteten Stammvarianten.

Die Stammvariante Wb 6-5 von Tolypocladium inflatum konnte durch Selektion von hochleistungsfähigen Monosporenlinien auf chemisch definierten Nährmedien, insbesondere durch die Anwesenheit von Kalzium- und Zinkionen im Medium, isoliert werden. Besonders überraschend ist die Isolierung dieser Stammvariante auch aufgrund der in der Literatur vertretenen Ansicht, daß die Cyclosporinbildung nur bei Anwesenheit von komplexen Nährstoffen wie Peptone oder bestimmten Aminosäuren in Größenordnungen erfolgen kann, die eine ökonomische Produktion erlauben. Die Selektante Wb 6-5 des Stammes Toly- pocladium inflatum wurde bei der DSM -Deutsche Sammlung von Mikroorganismen und Zellkulturen GmbH, D-38124 Braunschweig, Bundesrepublik Deutschland entsprechend dem Budapester Vertrag unter der Registriernummmer IMET 43899 hinterlegt.
Nach dem erfindungsgemäßen Verfahren wird die Selektante Wb 6-5 von Tolypocladium inflatum oder die Stammvariante F605 von Sesquicilliopsis rosariensis in einem Medium mit Ammoniumsalzen als einzig verfügbarer Stickstoffquelle sowie gegebenenfalls auch unter Zusatz von Zitrat und in Gegenwart ausgewählter zweiwertiger Kationen kultiviert. Darüber hinaus erwies es sich als vorteilhaft, daß man bei Fermentationen der einzelnen Stämme von rotbraun bis schwarzbraun-violett pigmentierten Emerskulturen ausgeht, die auf Mineralsalz- Glukose-Agar mit Ammoniumsalzen angezogen wurden, da diese verfahrensgemäß eine stabile Cyclosporin A-Produktion garantieren. Ferner ist es für die Selektante Wb 6-5 besonders günstig, in der Produktionskultur Ammoniumsulfat und/oder Diammoniumhydrogenphosphat als anorganische Stickstoffverbindungen zu verwenden. Die Zinkionenkonzentration sollte bei der Kultivierung dieses Stammes zweckmäßigerweise im Bereich von 0,5 mg/l bis 5,0 mg/l gewählt werden. Auffällig ist, daß die Produktivität der Selektante Wb 6-5 in überraschender Weise durch die Anwesenheit von Kaliumsalzen bzw. Kaliumionen negativ beeinflußbar ist. Diese Tatsache ist um so überraschender als alle für diesen Stamm publizierten Medien Kaliumsalze enthalten. Eine derartige Abhängigkeit konnte nicht für den Stamm F605 der Art Sesquicilliopsis rosariensis G.ARNOLD festgestellt werden. Für den letztgenannten Stamm ist vielmehr die Anwesenheit bestimmter zweiwertiger Metallionen im Fermentationsmedium von Bedeutung, insbesondere die Verfügbarkeit von Magnesium-, Eisen-, Zink- und Kalziumionen, die einen unmittelbaren Einfluß, wenn auch in unterschiedlichem Maße, auf die Syntheseaktivität ausüben. So sollte die Fermentation des Stammes F605 vorzugsweise bei einer Magnesiumkonzentration von 250 mg/l, einer Eisenkonzentration von 20 mg/l und einer Zinkkonzentration von 0,7 mg/l durchgeführt werden. Insbesondere ist es wichtig, auf eine ausgewogene Menge an Kalziumionen zu achten, wobei die Ionenkonzentration vorzugsweise bei Werten von 10 mg/l bis 200 mg/l liegen sollte, da bei Abwesenheit von Kalzium die Produktivität des Stammes F605 bei Einhaltung aller übrigen Bedingungen nicht effektiv ist. Gleichzeitig hat es sich für diese Stammvariante als ausgesprochen vorteilhaft erwiesen, die Produktbildungskultur in Gegenwart von Zitrationen im Bereich von 5 g/l bis 30 g/l, vorzugsweise bei 12 g/l durchzuführen.

Trotz der erkannten unterschiedlichen Bedürftigkeit der einzelnen Cyclosporin A-Produzentenstämme hinsichtlich des Gehaltes an bestimmten zweiwertigen Kationen im Produktionsmedium besteht jedoch für alle Stämme des vorliegenden Verfahrens der entscheidende verfahrenstechnische Fortschritt in einer Eliminierung der kostenintensiven, in ihrer Zusammensetzung heterogenen komplexen Stickstoffquelle. Dieses Ergebnis war insofern unerwartet, als durch AGATHOS et al. (J. Ind. Mikrobiol. Vol 1, 1986, 39-48 ) Ammonium und Nitrat als ungeeignet in Bezug auf die Cyclosporinproduktion erkannt wurde, weshalb sich diese Autoren für eine komplexe Stickstoffquelle als alleiniges N-haltiges Substrat entschieden haben. Auch die später publizierten Resultate mit anorganischen N-Quellen (MARGARITIS and CHAHAL, Biotechnol. Lett. Vol. 11, 1989, 765-768) bestätigen die obige Aussage.

Die Fermentation erfolgt nach einer ein- bzw. zweistufigen Vorzucht in der submersen Hauptkultur unter aeroben und sterilen Bedingungen, indem die unterschiedlichen Produzentenstämme in einem flüssigen Medium bei Temperaturen von 20 °C bis 30 °C sowie einer Azidität von 3,0 bis 7,5 für die Dauer von 5 bis 11 Tagen kultiviert werden.

Der Ablauf des gesamten Verfahrens läßt sich allgemein wie folgt charakterisieren:
Myzelrasenstücke aus Emerskulturen des Stammes Wb 6-5 bzw. auch deren Sporen werden in 0,9 %iger NaCl-Lösung suspendiert und auf einem Nähragar ausplattiert, welcher als Kohlenstoffquelle Mono- oder Disaccharide sowie Ammoniumsulfat als Stickstoffquelle enthält und für die Dauer von 14 bis 21 Tagen bei 24 bis 28 °C, vorzugsweise bei 24 °C, bebrütet wird. Die Anzucht des Stammes F605 erfolgt in gleicher Weise, jedoch ohne Ammoniumsulfat im Nähragar. Von den entstandenen Einzelkolonien werden die luftmyzelarmen, rot- bis schwarzbraun pigmentierten Exemplare ausgewählt und nach Übertragung auf Mineralsalz-Kohlenhydrat-Agar zur Herstellung von 2 bis 4 cm großen Myzelrasenarealen verwendet. Diese werden als Agarausstich in die erste submerse Vorzuchtpassage übertragen, die in 100 ml- bzw. 500 ml-Erlenmeyerkolben kultiviert wird, welche jeweils 20 % ihres Volumens als Anzuchtmedium enthalten. Die Nährlösung enthält Glukose, Maltose oder Saccharose als Kohlenstoffquelle, weiterhin organische oder anorganische Stickstoffsubstrate sowie Mineralsalze und wird nach der Beimpfung für die Dauer von 48 bis 96 Stunden bei 24 bis 28 °C unter Schütteln auf einem Rundschwinger bei 100 bis 240 rpm kultiviert. Nach Ausreifen der ersten Anzucht in Flüssigkultur (entspricht erste Vorkultur) erfolgt gegebenenfalls die Beimpfung einer zweiten Anzucht (entspricht zweite Vorkultur) mit einem Inokulum von 5 bis 10 % des Volumens der Nährlösung der Folgekultur. Diese Stufe besteht entweder aus 500 ml-Erlenmeyerkolben, die 100 ml des o.a. Mediums enthalten, oder aus gerührten und belüfteten Kleinfermentoren mit einem Nettovolumen von 5 bis 25 l bei üblichem technischem Ausstattungsgrad für Temperierung und Belüftung. Nach 48- bis 96-stündiger Kultivierung wird das Hauptkulturmedium, welches als alleinige Stickstoffquelle Ammoniumsalze enthält, mit einer Menge von 5 bis 10 % des Volumens mit der Vorkultur beimpft und für die Dauer von 5 bis 20 Tagen unter Rühren und Belüften bzw. durch Rundschütteln kultiviert. Unter diesen Bedingungen erreichen die Fermentationsansätze Leistungen von bis zu 3000 mg Cyclosporin A pro Liter, welches analytisch durch HPLC in bekannter Weise erfasst wird. Die Kultursuspensionen weisen zum Erntezeitpunkt eine rot- bis schwarzbraune Färbung auf. Das erfindungsgemäße Verfahren zur Isolierung von Cyclosporin A aus Kultursuspensionen besteht darin, daß man diese Kultursuspenspension mit einem Filterhilfsmittel versetzt, anschließend filtriert, die feuchte Biomasse trocknet, das so gewonnene Gemisch von feuchter Biomasse und Filterhilfsmittel mit einem niederen Carbonsäureester oder alternativ mit überkritischen Gasen, vorzugsweise CO₂ extrahiert, den Extrakt durch Verteilung zwischen wasserhaltigem Methanol und Petrolether entfettet und den so vorgereinigten Extrakt an Kieselgel und/oder Aluminiumoxid chromatographiert.
Die Vorteile dieser Isolierungsmethode liegen vor allem darin:
- verlustlose Abtrennung der Biomasse trotz geringer Teilchengröße, Entstehung eines trocknungsfähigen Materials, welches stabil, lagerfähig und extrahierbar ist
- Extraktionsverfahren mit geringem Anteil der mitgeführten Ballaststoffe bei vermindertem Reinigungsaufwand.
- Adaption der weiteren Reinigungsschritte an die speziellen Eigenschaften der verwendeten Produzenten
- Verringerung des Einsatzes von Extraktionsmittel

Als erschwerend erweist sich dabei die Tatsache, daß die Biomasse der verwendeten Produzenten nicht, wie üblich bevorzugt in Hyphenform, sondern in korpuskularer Form mit Partikelgrößen von 10-30 µm anfällt und schlecht filtrierbare, schwierig zu trennende Filterkuchen bildet. Erfindungsgemäß wird diese Schwierigkeit umgangen, indem die Kultursuspension direkt oder nach vorheriger Aufkonzentrierung mittels Zentrifugation mit einem Filterhilfsmittel ausreichender Trennwirkung (z.B. umkristallisierter Gips =Precosit®, Calcitmehl) versezt und anschließend filtriert wird. Ein Masseverhältnis Filterhilfsmittel /Biotrockenmasse von (1...4):1 gewährleistet gute Filtrierbarkeit, schnelle Trocknung und effektive Rückstandsabtrennung nach der Extraktion.
Die so gewonnene feuchte Biomasse, die noch zwischen 35 und 60 Masse % Kulturfiltrat enthält, läßt sich überraschenderweise trotz ihrer Peptidnatur praktisch ohne Substanzverlust durch Konvektionstrocknung (z.B. Vakuumtrockenschrank, Umlufttrockenschrank, Wirbelschicht- oder Bandtrockner) bei Temperaturen bis 80 °C, vorzugsweise bei 40 - 60°C in ein lagerfähiges Trockenmycelpräparat überführen, dem zweckmäßig eine Masseverhältnis Feuchte :Feststoff von (0,02 ....0,15):1 belassen wird. Eine Alternative besteht in der Lyophilisierung, die ebenfalls ein stabiles und extrahierbares Trockenprodukt liefert.
Für die nachfolgende zwei- bis dreimalige Extraktion kommt vorzugsweise Essigsäureethylester in Betracht, der bedeutend weniger Ballststoffe mitführt als beispielsweise Ethanol oder Aceton. Dies gilt insbesondere dann, wenn die Anzahl der Extraktionen auf 2 beschränkt wird.
Der nach der Eindampfung verbleibende Rückstand wird in an sich bekannter Weise durch Verteilung zwischen wasserhaltigem Methanol oder Ethanol und Petrolether teilentfettet und enthält bereits einen Substanzanteil um 40 Masse %. Ein Anfeuchten des Mycels vor der Extraktion mit Ammoniakwasser kann zweckmäßig sein. Bei Verarbeitung schwer extrahierbarer Myceltypen wird das Mycel vor der Extraktion mit 20 bis 40 Masse % vorzugsweise 25 Masse %,über 8 bis 72 Stunden vorgequollen und die erste Extraktion mit einem Ultra-Turrax vorgenommen.
Alternativ zur fest-flüssig Extraktion mit organischen Lösungsmitteln kann der Wirkstoff auch mit überkritischen Gasen, vorzugsweise mit CO_{2,} aus dem Trockenmycel extrahiert werden. Druck und Temperatur sind gasspezifisch. Für CO₂ ergibt sich ein sinnvoller Bereich von ca. 100 bis 300 bar und 35 bis 60°C. Zweckmäßgerweise wird die Extraktion durch Zusatz eines

Schleppmittels unterstützt, z.B. 0,5 bis 1,5 Masse % Ethanol zum CO_{2.} Verunreinigungen, vor allem Fette, können mittels mehrstufiger Extraktion bzw. fraktionierter Abscheidung abgetrennt werden.
Die erste säulenchromatographische Reinigung erfolgt wahlweise an Aluminiumoxid neutral (50 bis 100-fache Menge) oder Kieselgel (30 -bis 60-fache Menge) mit Essigsäureethylester als mobiler Phase, die zweite Reinigung erfolgt immer an Kieselgel, vorzugsweise der 40-fachen Menge in Ethylacetat.
In logischer Fortsetzung der überkritischen Extraktion ist alternativ zur Normaldruck-Flüssigkeits-Chromatographie die weitere Wirkstoffselektion mittels HPLC möglich.

### Ausführungsbeispiele

### Beispiel 1

Myzelrasenstücke von Oberflächenkulturen des Stammes Tolypocladium inflatum Wb 6-5 werden zunächst auf Glukose-Mineralsalz-Agar übertragen, der folgende Zusammensetzung aufweist (g/l): Glukose 20;
(NH4)₂SO₄ 5; KH₂PO4 2; MgSO₄*7H₂O 0,5; CaCO₃ 3,4; Agar 20; pH-Wert 5,3 bis 5,7; Sterilisation 30 min bei 120 °C. Nach einer Bebrütungszeit der Petrischalen bei 24 °C für die Dauer von 14 bis 21 Tagen werden von den entstandenen Arealen die rot- und schwarzbraun pigmentierten ausgewählt. Die Beimpfung der ersten Submersvorkultur erfolgt durch Übertragung eines ca. 1cm² großen Rasenstückes auf 100 ml des Anzuchtmediums folgender Zusammensetzung (g/l): Glukose 50; Caseinpepton 10; KH₂PO₄ 1; KCl 2,5 ad 1 l destilliertes Wasser; pH-Wert 5,3 bis 5,6; Sterilisation 30 min bei 120 °C. Die 500 ml-Anzuchtkolben werden 72 Stunden bei 24 bis 25 °C mit 180 bis 220 rpm auf Rundschüttlern kultiviert, sie zeigen als Reifekriterium eine braunschwarze Färbung und dienen in einer Menge von 5% als Impfmaterial des Hauptkulturmediums, welches zu je 100 ml in 500 ml-Erlenmeyerkolben abgefüllt folgende Zusammensetzung aufweist (g/l): Glukose 80; (NH4)₂SO4 5; (NH₄)₂HPO₄ 2; MgSO₄*7 H₂O 0,5; ZnSO₄ * 7 H₂O 0,008; CaCO₃ 5,1; destilliertes Wasser ad 1 l; pH-Wert 5,3 bis 5,9; Sterilisation 30 min bei 120 °C. Die Kultivierung erfolgt 11 Tage bei 24 bis 25 °C auf einem Rundschwinger mit 180 rpm.
Nach Beendigung der Fermentation wird die Kulturbrühe mit Methanol im Verhältnis 5:1 (v/v) versetzt und der Extrakt zur Wertbestimmung verwendet. Diese erfolgt in an sich bekannter Weise durch Beschickung von HPLC-Säulen. Die Leistung derartiger Fermentationsansätze beträgt 1100 mg Cyclosporin A pro Liter.

### Beispiel 2

Von einer Emerskultur des Stammes Sesquicilliopsis rosariensis F605 auf Malzextraktagar (20 g Malzextrakt/l) wird nach mindestens 14-tägiger Bebrütung bei 24 °C eine Sporensuspension hergestellt, die zur Beimptung einer Vorkultur dient. Das Vorkulturmedium hat folgende Zusammensetzung (g/l): Maltose oder Glukose 75; Caseinpepton 25; KH₂PO₄ 1; KCl 2,5; entionisiertes Wasser ad 1 l; pH-Wert 5,5; Sterilisation 30 min bei 120 °C. 100 ml Vorkulturmedium werden mit 2 * 10⁷ Sporen beimpft und in 500 ml-Erlenmeyerkolben bei 24 °C für 72 Stunden auf einem Rundschwinger mit einer Frequenz von 190 rpm bebrütet. Anschließend erfolgt die Übertragung der Kultur in ein Hauptkulturmedium im Verhältnis 1:10, welches sich wie folgt zusammensetzt (g/l):Glukose 100; Diammoniumhydrogenzitrat 15; KH₂PO4 1; MgSO4*7 H₂O 2, 5; FeSO₄ _{*}7H₂O 0,1; ZnSO₄ *7 H₂O 0,003; entionisiertes Wasser ad 1 l; pH-Wert 5,5; Sterilisation 60 min bei 110 °C. Alle Kulturbedingungen werden entsprechend der Vorkultur gewählt. Der am 11.Kulturtag ermittelte Gehalt an Cyclospo- rin A betrug maximal 1150 mg/l Kultursuspension. Die Bestimmung des Wirkstoffes erfolgte wie unter Beispiel 1 beschrieben.

### Beispiel 3

Anzucht und Kultivierung des Stammes F605 erfolgt in der Vorkultur wie im 2. Ausführungsbeispiel beschrieben, jedoch wird im Gegensatz dazu der Produzentenstamm nach der Vorkultur in einen 5 l-Laborfermentor überführt, welcher das bereits im 2. Ausführungsbeispiel vorgestellte Hauptkulturmedium enthält und mit 200 bis 500 min⁻¹
gerührt und mit einem Volumenstrom von 0,25 bis 1,0 *min⁻¹
*1⁻¹ belüftet wird. Die bis zu 14 Tagen durchgeführte Fermentation ergab Cyclosporin A-Ausbeuten von maximal 3150 mg/l.
gerührtgerührt und mit einem belüftet wird. Die bis zu 14 Tagen ntation ergab Cyclosporin A-Ausbeuten von maximal 3150 mg/l.

### Beispiel 4

Nach einer entsprechend Ausführungsbeispiel 2 durchgeführten Impfmaterialanzucht für den Stamm F605 wird ein Rührfermentor mit folgender Nährlösung (g/l) : Glukose 50; Sojaschrot 2,5; (NH₄)₂SO₄ 4,1; KH₂PO₄ 5,0; pH-Wert 5,5; Sterilisation 60 min 110 °C; mit 2 * 10⁸ Sporen pro Liter Medium beimpft. Temperatur, Rührung und Belüftung entsprechen den im 3.Ausführungsbeispiel für die Hauptkultur gemachten Angaben. Die so in 4 Tagen erhaltene Kultur dient als 10 %iges Inokulum für die Produktionsstufe in einem 450 l-Fermentor aus legiertem Stahl, welcher die im 2. Ausführungsbeispiel angegebene Hauptkulturnährlösung enthält und unter Einhaltung der oben für die Vorkultur genannten Bedingungen kultiviert wird.
In Fermentationen, die in der angegebenen Art durchgeführt wurden, hatten durchschnittlich nach 14 Kulturtagen 2 g Cyclosporin A pro Liter Kultursuspension gebildet.

### Beispiel 5

6 l Kultursusupension des Stammes F605 der Spezies Sesquicilliopsis rosariensis G.ARNOLD (IMET 43 887) werden über eine mit Precosit® beschichtete Handfilterplatte gezogen, und das abgenommene Feuchtprodukt wird im Umlufttrockenschrank 6 Stunden bei 50°C getrocknet.
Man erhält 246 g Trockenmycel-Precosit® -Gemisch, das etwa 50 Masse % Precosit® enthält.
Das Gemisch wird mit 60 ml 10%igem wäßrigem Ammoniak 15 min vorgequollen und dreimal mit insgesamt 4,2l Essigsäureethylester in 3 Portionen je 30 min gerührt.Der vom Feststoff abfiltrierte Geamtextrakt hinterläßt nach Abdestillieren des Lösungsmittels 21 g Rückstand, der durch Aufnehmen in 100 ml Ethanol, das 5% Wasser enthält, dreimaligen Ausschütteln mit je 300 ml Petrolether und Abdampfen des Methanols auf 14 g vermindert wurde.

Die säulenchromatographische Reinigung an 1000 g Aluminium-oxid neutral, Aktivitätsstufe l, in Essigsäureethylester lie-fert nach ca. 500 ml Vorlauf 1450 ml Hauptfraktion, nach Rechromatographie des Rückstandes an Kieselgel 60 Merck (0.063-0,2 mm) im gleichen Lösungsmittel und Verrühren mit der 7-fachen Menge n-Hexan erhält man 5,2g fast weißes Produkt mit 99 Masse% Cyclosporin A laut HPLC.

### Beispiel 6

Ein Volumen von 327,5 l Kultursuspension der im Beispiel 5 genannten Mutante mit einem Anteil von 21,2 % Biotrockenmasse wird mit 13,1 kg Precosit® vermischt und in 2 Teilchargen in einem Druckfilter mit einer Filterfläche von 0,5 m² Zentrifugalscheibenfilter, Bauart SEITZ) filtriert. Die Filterfläche wurde vorher mit einer PRECOSIT-Grundanschwemmung von 250 g pro Teilcharge bedeckt. Der feuchte Filterkuchen (Masse 28,5 kg) wird in einem Wirbelschichttrockner bei Zulufttemperaturen von 35 bis 55 °C getrocknet, so daß ein Trockenmycel-PRECOSIT-Gemisch mit einer Masse von 15,4 kg entsteht. Die erhaltenen Trockenmasse wird in einer Rührnutsche zunächst mit 100 l, dann mit 70 l Essigsäureethylester je 30 min gerührt. Die geklärten und vorgereinigten Extrakte konzentriert man erst im Vakuum-Umlaufverdampfer, dann im Vakuumrotationsverdampfer bis auf einen öligen Rückstand, der in 8 l Methanol aufgenommen wird. Nach Zusatz von 0,4 l Wasser wird dreimal mit je 16 l Petrolether ausgerührt. Die abgetrennte Methanolphase hinterläßt nach dem Einengen im Vakuumrotationsverdampfer 600 g Rückstand.

Die erste säulenchromatographische Reinigung an 24 kg Kieselgel 60 Merck in Essigsäureethylester liefert 275 g eines 79 masseprozentigen Rohcyclosporins. Die Rechromatographie er-folgt an 14 kg Kieselgel 60 Merck in Methylenchlorid mit 2,5 Masse % Methanolanteil.
Ausbeute: 102 g, Gehalt (HPLC) 97,5 Masse % Cyclosporin A

### Beispiel 7

Ein Vakuum-Drehzellenfilter mit 0,3 m² Filterflache wird mit 10 kg PRECOSIT® als Primäranschwemmung beschichtet. Darüber filtriert man 290 l einer Kultursuspension der im Beispiel 5 beschriebenen Mutante. Der Kultursuspension werden zuvor noch 60 g/l PRECOSIT® zugestzt. Man erhält 62,5 kg feuchten Filterkuchen mit 55,4 % Wassergehalt. 50 kg dieser Feuchtmasse werden in der Wirbelschicht getrocknet und liefern 22,5 kg Trockenmycel-PRECOSIT®-Gemisch mit einem Wirkstoffgehalt von 6,3 g /kg.

### Beispiel 8

20 kg des nach Beispiel 7 gewonnenen Trockenmycel-PRECOSIT®-Gemisches werden mit 5 l Methanol befeuchet und in einem verschlossenen Gefäß 24 Stunden bei Raumtemperatur stehengelassen. Danach trägt man das Material in 50 l Ethylacetat ein und turbiniert 40 min mit dem Ultra-Turrax®. Nach dem Abnutschen wird der Filterrückstand mit weiteren 50 l Ethylacetat 10 min gerührt und abgesaugt. Den Rückstand der vereinigten Extrakte verarbeitet man analog Beispiel 6 und erhält so 61,5 g Reincyclosporin A.

### Beispiel 9

0,52 kg des nach Beispiel 7 gewonnenen Trockenmycel-Precosit-Gemisches (TMP) werden mit 5,2 bis 6,5 kg/h überkritischem CO₂ bei 300 bar und 40°C unter Zusatz von 50 bis 80 ml Ethanol über eine Zeit von 6 h extrahiert. Es werden ca. 60% des im TMP enthaltenen Cyclosporin A extrahiert. Der so erhaltene Extrakt wird analog zu Beispiel 6 zur Reinsubstanz weiterverarbeitet.

## Patentansprüche

1. Verfahren zur fermentativen Herstellung und Isolierung von Cyclosporin A durch aerobe Fermentation eines pilzlichen Mikroorganismus in einem Medium mit Stickstoff- und Kohlenstoffsubstraten sowie Mineralsalzen , dadurch gekennzeichnet, daß man zur Fermentation einen neuen Stamm der Pilzspezies Sesquicilliopsis rosariensis G.ARNOLD oder eine durch Mutagenese und/oder Selektion daraus erhaltene Stammvariante als Produzentenstamm verwendet, in einem Medium, welches Ammoniumsalze als einzige Stickstoffquelle, eine Kohlenstoffquelle und ggf. Zitrat sowie 2-wertige Kationen enthält, kultiviert, die Kultursuspension mit einem Filterhilfsmittel versetzt, anschließend filtriert, das so gewonnene Gemisch von feuchter Biomasse und Filterhilfsmittel trocknet, mit einem niederen Carbonsäureester oder einem überkritischem Gas vorzugsweise CO₂ extrahiert, den Extrakt ggf. durch Verteilung zwischen wasserhaltigem Methanol und Petrolether entfettet und den so vorgereinigten Extrakt in an sich bekannter Weise an Kieselgel und oder Aluminiumoxid bzw. durch präparative HPLC chromatographiert.

2. Verfahren nach Anspruch 1 dadurch gekennzeichnet, daß man den neuen Stamm der Pilzspezies Sesquicilliopsis rosariensis G.ARNOLD IMET 43 888 verwendet.

3. Verfahren nach Anspruch 1 dadurch gekennzeichnet, daß man den neuen Stamm der Pilsspezies Sesquicilliopsis rosariensis G.ARNOLD F605, IMET 43 887 verwendet.

4. Pilzstamm Sesquicilliopsis rosariensis G.ARNOLD IMET
Nr. 43 888

5. Pilzstamm Sesquicilliopsis rosariensis F 605 G.ARNOLD IMET 43 887

6. Verfahren nach Anspruch 1, dadurch gekennzeichnet, daß man von rot-bis schwarzbraun pigmetierten Emerskulturen ausgeht, die auf einem Mineralsalz-Glucose-Agar mit Ammoniumsalzen als einziger Stickstoffquelle angezogen wurden.

7. Verfahren nach Anspruch 1, dadurch gekennzeichnet, daß man die Fermentation in Gegenwart von Zink-, Eisen- und oder Kalziumionen durchführt.

8. Verfahren nach Anspruch 1, dadurch gekennzeichnet, daß als Filterhilfsmittel umkristallisierter Gips vorzugsweise Calcitmehl verwendet wird.

9. Verfahren nach Anspruch 1 ,dadurch gekennzeichnet, daß als Extraktionsmittel für das getrocknete Biomasse -Filterhilfsmittel-Gemisch Essigsäureethylester oder überkritische
Gase, vorzugsweise CO₂ eingesetzt werden.

10. Verfahren nach Anspruch 1 und 9, dadurch gekennzeichnet, daß das getrockneten Biomasse-Filterhilfsmittel-Gemisch
vor der Extraktion mit Methanol oder wäßrigem Ammoniak vorgequollen wird.

## Claims

1. Process for the fermentative production and isolation of cyclosporin A by aerobic fermentation of a fungal microorganism in a medium containing nitrogen substrate, carbon substrate and mineral salts, characterized in that a novel strain of the fungal species Sesquicilliopsis rosariensis G. ARNOLD or a variant strain obtained therefrom by mutagenesis and/or selection is used as producer strain for the fermentation, cultured in a medium which contains ammonium salts as sole nitrogen source, a carbon source and if appropriate citrate and divalent cations, a filtration aid is added to the culture suspension which is then filtered and the mixture thus obtained of moist biomass and filtration aid is then dried, extracted with a lower carboxylic ester or a supercritical gas, preferably CO₂, the extract is defatted if necessary by partition between aqueous methanol and petroleum ether and the extract thus prepurified is chromatographed in a manner known per se on silica gel and/or alumina or by preparative HPLC.

2. Process according to Claim 1, characterized in that the novel strain of the fungal species Sesquicilliopsis rosariensis G. ARNOLD IMET 43 888 is used.

3. Process according to Claim 1, characterized in that the novel strain of the fungal species Sesquicilliopsis rosariensis G. ARNOLD F605, IMET 43 887 is used.

4. Fungal strain Sesquicilliopsis rosariensis G. ARNOLD IMET No. 43 888.

5. Fungal strain Sesquicilliopsis rosariensis F 605 G. ARNOLD IMET 43 887.

6. Process according to Claim 1, characterized in that surface cultures pigmented from red to dark brown are used as starting material which were cultured on mineral salt-glucose agar containing ammonium salts as sole nitrogen source.

7. Process according to Claim 1, characterized in that the fermentation is carried out in the presence of zinc ions, iron ions and/or calcium ions.

8. Process according to Claim 1, characterized in that the filtration aid used is recrystallized gypsum, preferably calcite flour.

9. Process according to Claim 1, characterized in that the extraction medium used for the dried biomass/filtration aid mixture is ethyl acetate or supercritical gases, preferably CO₂.

10. Process according to Claim 1 and 9, characterized in that the dried biomass/filtration aid mixture is preswollen with methanol or aqueous ammonia before the extraction.

## Revendications

1. Procédé de production par fermentation et d'isolement de la cyclosporine A, par culture par fermentation aérobie d'un micro-organisme fongique dans un milieu contenant des substrats azotés et carbonés ainsi que des sels minéraux caractérisé en ce que l'on utilise comme souche productrice pour la fermentation une nouvelle souche de l'espèce de champignon *Sesquicilliopsis rosariensis* G. ARNOLD ou un variant obtenu à partir de celle-ci par mutagénèse et/ou sélection, on la cultive dans un milieu qui contient des sels d'ammonium en tant que seule source d'azote, une source de carbone et éventuellement un citrate ainsi que des cations divalents, on ajoute un adjuvant de filtration à la suspension de culture, puis on la filtre, on sèche le mélange, ainsi obtenu, de biomasse humide et d'adjuvant de filtration, on l'extrait à l'aide d'un ester d'acide carboxylique inférieur ou d'un gaz supercritique, CO₂ de préférence, on élimine éventuellement les matières grasses de l'extrait par partage entre du méthanol aqueux et de l'éther de pétrole, et l'extrait ainsi purifié au préalable est chromatographié d'une façon connue en soi, sur du gel de silice et/ou de l'oxyde d'aluminium, ou bien par HPLC préparative.

2. Procédé selon la revendication 1, caractérisé en ce que l'on utilise la nouvelle souche de l'espèce de champignon *Sesquicilliopsis rosariensis* G. ARNOLD IMET 43 888.

3. Procédé selon la revendication 1, caractérisé en ce que l'on utilise la nouvelle souche de l'espèce de champignon *Sesquicilliopsis rosariensis* G. ARNOLD F605, IMET 43 887.

4. Souche de champignon *Sesquicilliopsis rosariensis* G. ARNOLD IMET 43 888.

5. Souche de champignon *Sesquicilliopsis rosariensis* F605 G. ARNOLD, IMET 43 887.

6. Procédé selon la revendication 1, caractérisé en ce que l'on part de cultures émergées pigmentées en rouge à marron-noir, qui ont été cultivées sur une gélose au glucose-sels minéraux avec des sels d'ammonium en tant que seule source d'azote.

7. Procédé selon la revendication 1, caractérisé en ce que la fermentation est effectuée en présence d'ions zinc, fer et/ou calcium.

8. Procédé selon la revendication 1, caractérisé en ce que l'on utilise comme adjuvant de filtration du gypse recristallisé, de préférence de la poudre de calcite.

9. Procédé selon la revendication 1, caractérisé en ce que, comme agent d'extraction pour le mélange séché de biomasse et d'adjuvant de filtration, on utilise de l'acétate d'éthyle ou des gaz supercritiques, CO₂ de préférence.

10. Procédé selon les revendications 1 et 9, caractérisé en ce que, avant l'extraction, le mélange séché de biomasse et d'adjuvant de filtration est prégonflé avec du méthanol ou de l'ammoniaque.
